# EUROPÄISCHE PATENTSCHRIFT

(11) **EP 4 346 979 B1**
(45) Veröffentlichungstag und Bekanntmachung des Hinweises auf die Patenterteilung: **13.08.2025**
(21) Anmeldenummer: 22729737.1
(22) Anmeldetag: 01.06.2022
(51) Int. Cl.: A61B 17/34, A61B 10/00, A61M 25/06, A61B 17/00

(54) **KANÜLENANSATZ FÜR EINE SPINALKANÜLE**
NEEDLE ATTACHMENT FOR A SPINAL NEEDLE
FIXATION D'AIGUILLE POUR AIGUILLE SPINALE

(30) Priorität: 02.06.2021 DE 102021205601
(43) Veröffentlichungstag der Anmeldung: 10.04.2024
(73) Patentinhaber: B. Braun Melsungen AG, 34212 Melsungen (DE)
(72) Erfinder: KOPP, Florin, 26419 Schortens (DE); RIEKEN, Franziska, 34128 Kassel (DE); SCHLEMME, Dominik, 34212 Melsungen (DE)
(74) Vertreter: Patentanwälte Ruff, Wilhelm, Beier, Dauster & Partner mbB
(86) Internationale Anmeldenummer: PCT/EP2022/064893
(87) Internationale Veröffentlichungsnummer: WO 2022/253892

(56) Entgegenhaltungen:
- EP-A1- 0 522 737
- US-A1- 2002 055 715
- US-A1- 2011 071 480

## Beschreibung

Die Erfindung betrifft einen Kanülenansatz für eine Spinalkanüle, aufweisend einen zwischen einem proximalen Ende und einem distalen Ende erstreckten Körper, einen durch den Körper erstreckten Fluidkanal mit einem Beobachtungskanalabschnitt, der durch einen transparenten Bereich des Körpers hindurch von außen einsehbar ist, und aufweisend wenigstens eine in dem transparenten Bereich angeordnete optische Linse, mittels derer der Beobachtungskanalabschnitt optisch vergrößert einsehbar ist.

Spinalkanülen werden beispielsweise im Rahmen einer Spinalanästhesie zur Punktion des Rückenmarks verwendet und weisen einen Kanülenansatz und eine an diesem festgelegte Hohlnadel auf. In der Anwendung wird die Hohlnadel in den mit Cerebrospinalflüssigkeit (CSF bzw. Liquor) gefüllten Liquorraum des Rückenmarks vorgeschoben. Um die korrekte Lage der Hohlnadel zu bestätigen, wird der hierbei in den Kanülenansatz eintretende Flüssigkeitsrückfluss (englisch: flashback) optisch kontrolliert.

Ein zur optischen Kontrolle eingerichteter Kanülenansatz ist aus der US 6 656 161 B2 bekannt und weist einen Körper auf, an dessen distalem Ende eine Hohlnadel festlegbar ist. Zwischen dem distalen Ende und einem proximalen Ende des Körpers ist ein Fluidkanal erstreckt. Der Fluidkanal weist einen als Kammer bezeichneten Beobachtungskanalabschnitt auf. Eine an die Kammer angrenzende Wandung des Körpers ist mit einer optischen Linse versehen. Hierdurch soll die Kammer für einen Beobachter von außen optisch vergrößert wahrnehmbar sein. Dies soll die Erkennung des Flüssigkeitsrückflusses verbessern. Zudem lehrt die besagte Patentschrift, dass die Kammer ein möglichst kleines Volumen aufweisen soll.

Ein weiterer zur optischen Kontrolle eingerichteter Kanülenansatz ist aus der US 2011/071480 A1 bekannt. Auch dieser bekannte Kanülenansatz weist einen Körper mit einem Beobachtungskanalabschnitt auf, der durch einen transparenten Bereich des Körpers hindurch von außen einsehbar ist. In dem Beobachtungskanalabschnitt ist ein optisches Prisma angeordnet.

Weiter ist aus der EP 0 522 737 A1 ein Kanülenansatz mit einer konvexen Linse bekannt, die als Vergrößerungsglas zur besseren Erkennbarkeit von in dem Kanülenansatz befindlicher Flüssigkeit dient.

Aufgabe der Erfindung ist es, einen Kanülenansatz der eingangs genannten Art bereitzustellen, der eine verbesserte optische Kontrolle des Flüssigkeitsrückflusses ermöglicht.

Die vorliegende Erfindung wird im unabhängigen Anspruch 1 definiert. Die weiteren Ausführungsformen werden in den abhängigen Ansprüchen definiert.

Diese Aufgabe wird dadurch gelöst, dass wenigstens ein optisches Prisma in dem Beobachtungskanalabschnitt vorhanden ist, das zur Brechung und/oder Reflexion von durch die optische Linse hindurch in den Beobachtungskanalabschnitt einfallendem Licht eingerichtet ist. Durch die das Licht brechenden und/oder reflektierenden optischen Eigenschaften des wenigstens einen Prismas ergeben sich für einen Beobachter deutlich unterschiedliche optische Wahrnehmungen und/oder Bilder, je nachdem, ob der Beobachtungskanalabschnitt mit Luft oder mit Flüssigkeit gefüllt ist. Optische Unterschiede stellen sich zwar grundsätzlich auch ohne das wenigstens eine Prisma ein. Allerdings treten die optischen Unterschiede ohne Prisma in deutlich geringerem Umfang zutage und sind daher für den Beobachter schwieriger wahrnehmbar. Durch das wenigstens eine optische Prisma kann somit sehr deutlich festgestellt werden, ob bereits ein Flüssigkeitsrückfluss in dem Beobachtungskanalabschnitt stattfindet oder nicht. Dabei haben die Erfinder erkannt, dass durch die Kombination der optischen Vergrößerung einerseits und der Lichtbrechung und/oder Reflexion andererseits eine selbst bei widrigen Lichtverhältnissen besonders zuverlässige und einfache optische Kontrolle ermöglicht wird. Der Kanülenansatz kann auch als Kanülenhub oder kurz Hub bezeichnet werden. Das distale Ende des Körpers ist zur Verbindung mit einer Hohlnadel der Spinalkanüle eingerichtet. Vorzugsweise ist das in Längsrichtung des Körpers gegenüberliegende proximale Ende zur Verbindung mit einer weiteren medizinischen Komponente, beispielsweise einer Spritze, einer medizinischen Schlauchleitung oder dergleichen, eingerichtet. Der Flüssigkeitsrückfluss erfolgt in proximaler Richtung. Bei einer Ausgestaltung ist das wenigstens eine optische Prisma ein von dem Körper separat gefertigtes und hiernach in den Beobachtungskanalabschnitt eingefügtes Bauteil. Bei anderen Ausgestaltungen ist das wenigstens eine optische Prisma einstückig in den Körper integriert. Entsprechendes gilt sinngemäß im Hinblick auf die wenigstens eine optische Linse. Dementsprechend ist die wenigstens eine optische Linse bei einer Ausgestaltung als separates Bauteil gefertigt und hiernach an dem Körper befestigt. Bei anderen Ausgestaltungen ist die wenigstens eine optische Linse einstückig in den Körper integriert. Der Körper ist wenigstens im Bereich des Beobachtungskanalabschnitts, der wenigstens einen optischen Linse und/oder dem wenigstens einen optischen Prisma transparent. Hierfür ist der Körper wenigstens abschnittsweise aus einem transparenten Werkstoff, vorzugweise einem Kunststoff, gefertigt.

In Ausgestaltung der Erfindung sind die wenigstens eine optische Linse und das wenigstens eine optische Prisma durch ein- und dieselbe transparente Wandung des Körpers ausgebildet und somit einstückig zusammenhängend, wobei die wenigstens eine optische Linse einer Außenseite der transparenten Wandung und das wenigstens eine optische Prisma einer Innenseite der transparenten Wandung zugeordnet sind. Hierdurch wird insbesondere ein vereinfachter Aufbau des Kanülenansatzes erreicht. Dies vereinfacht die Herstellung und Montage. Durch die in den Körper, genauer: dessen transparente Wandung, integrierte Bauweise der wenigstens einen optischen Linse und des wenigstens einen optischen Prismas kann die erforderliche Anzahl an Bauteilen des Kanülenansatzes verringert werden. Die Außenseite der transparenten Wandung ist dem Beobachtungskanalabschnitt in radialer Richtung abgewandt. Die Innenseite der transparenten Wandung ist dem Beobachtungskanalabschnitt in radialer Richtung zugewandt. Die transparente Wandung des Körpers ist in dessen transparentem Bereich angeordnet oder bildet den transparenten Bereich des Körpers aus. Die Außenseite der transparenten Wandung grenzt an eine Umgebung. Die Innenseite der transparenten Wandung grenzt an den Beobachtungskanalabschnitt.

In weiterer Ausgestaltung der Erfindung weist der Fluidkanal ein Gesamtvolumen auf, wobei der Beobachtungskanalabschnitt einen überwiegenden Volumenanteil des Gesamtvolumens einnimmt. Mit anderen Worten ist der Beobachtungskanalabschnitt möglichst groß im Vergleich zu den übrigen Abmessungen des Fluidkanals und/oder des Körpers. Dementsprechend nimmt der Beobachtungskanalabschnitt den überwiegenden Volumenanteil des Gesamtvolumens des Fluidkanals ein. Durch die vergleichsweise große Dimensionierung des Beobachtungskanalabschnitts kann eine relativ niedrige Fließgeschwindigkeit des Flüssigkeitsrückflusses erreicht werden. Dies in Relation zu einem vergleichsweise klein dimensionierten Beobachtungskanalabschnitt. Durch die verlangsamte Fließgeschwindigkeit verbleibt einem Beobachter mehr Zeit zur optischen Wahrnehmung eines sich einstellenden Flüssigkeitsrückflusses. Die Erfinder haben erkannt, dass die verminderte Fließgeschwindigkeit mit einer nochmals verbesserten optischen Kontrolle einhergeht. Durch die schnelle Sichtbarkeit des Flüssigkeitsrückflusses kann der Beobachter schneller reagieren, so dass weniger Flüssigkeit aspiriert werden muss. Zudem kann vermieden werden, dass CSF aus dem Kanülenansatz heraustropft. Folglich kann auch vermieden werden, dass der Flüssigkeitsrückfluss erst durch ein Heraustropfen der Flüssigkeit erkannt wird und hierdurch ein Flüssigkeitsverlust für den Patienten entsteht. Im Ergebnis können gesundheitliche Beeinträchtigungen des Patienten vermieden werden. Insbesondere wird das Risiko eines sogenannten Post Dural Puncture Headache (PDPH) verringert.

In weiterer Ausgestaltung der Erfindung weist der Fluidkanal einen maximalen Innendurchmesser auf, der maximal 45 %, bevorzugt maximal 10 %, größer ist als ein maximaler Innendurchmesser des Beobachtungskanalabschnitts. Mit anderen Worten weist der Beobachtungskanalabschnitt bei dieser Ausgestaltung in Relation zu den übrigen Durchmesserabmessungen des Fluidkanals einen möglichst großen Innendurchmesser auf. Vorzugsweise ist der Innendurchmesser des Fluidkanals über dessen Längserstreckung veränderlich. Dementsprechend weist der Fluidkanal im Durchmesser relativ enge und relativ weite Kanalabschnitte auf. Der Beobachtungskanalabschnitt ist im Durchmesser möglichst weit. Bei einer Ausgestaltung bildet der maximale Innendurchmesser des Beobachtungskanalabschnitts gleichsam den maximalen Innendurchmesser des gesamten Fluidkanals. Durch den relativ großen maximalen Innendurchmesser des Beobachtungskanalabschnitts wird eine relativ geringe Fließgeschwindigkeit des Flüssigkeitsrückflusses erreicht. Zu den hiermit einhergehenden Vorteilen wird auf die Erläuterungen in Zusammenhang mit der vorhergehenden Ausgestaltung verwiesen. Das dort Gesagte gilt sinngemäß auch für diese Ausgestaltung der Erfindung. Der Innendurchmesser kann auch als hydraulisch wirksamer oder hydraulischer Durchmesser bezeichnet werden und insoweit die Dimension einer Fläche aufweisen.

In weiterer Ausgestaltung der Erfindung weist das proximale Ende des Körpers einen genormten Fluidkonnektor mit einem genormten Innendurchmesser auf, wobei der maximale Innendurchmesser des Beobachtungskanalabschnitts maximal 45 %, bevorzugt maximal 10 %, kleiner ist als der genormte Innendurchmesser des Fluidkonnektors. Auch diese Ausgestaltung weist einen vergleichsweise großen Innendurchmesser des Beobachtungskanalabschnitts auf, wobei der Innendurchmesser in Relation zu dem genormten Innendurchmesser des genormten Fluidkonnektors definiert ist. Auch diese Ausgestaltung ermöglicht eine möglichst geringe Fließgeschwindigkeit des Flüssigkeitsrückflusses innerhalb des Beobachtungskanalabschnitts. Zu den hiermit einhergehenden Vorteilen wird auf das zuvor Gesagte verwiesen. Der genormte Fluidkonnektor ist bei einer Ausgestaltung ein NRFit-Konnektor nach DIN EN ISO 80369-6, wobei der maximale Innendurchmesser des Beobachtungskanalabschnitts maximal 20 %, bevorzugt maximal 15 %, besonders bevorzugt maximal 10%, kleiner ist als der genormte Innendurchmesser des NRFit-Konnektors. Bei einer anderen Ausgestaltung ist der Fluidkonnektor ein Luer-Konnektor nach DIN EN ISO 80369-7, wobei der maximale Innendurchmesser des Beobachtungskanalabschnitts maximal 45 %, bevorzugt maximal 30 %, kleiner ist als der genormte Innendurchmesser des Luer-Konnektors.

In weiterer Ausgestaltung der Erfindung sind wenigstens zwei optische Prismen und wenigstens zwei optische Linsen vorhanden, die paarweise als Linsen-Prismen-Paare in Umfangsrichtung des Beobachtungskanalabschnitts zueinander winkelversetzt angeordnet sind. Durch die winkelversetzte Anordnung mehrerer Linsen-Prismen-Paare kann die optische Kontrolle durch einen Beobachter in unterschiedlichen Beobachtungswinkeln relativ zu dem Körper erfolgen. Mit anderen Worten kann der Beobachter den Flüssigkeitsrückfluss nicht lediglich in einer einzigen auf den Körper gerichteten Blickrichtung, sondern in unterschiedlichen Blickrichtungen kontrollieren. Hierdurch wird die optische Kontrolle nochmals verbessert. Sofern genau zwei Linsen-Prismen-Paare vorhanden sind, sind diese vorzugsweise um 90° oder 180° zueinander winkelversetzt angeordnet. Bei einem Winkelversatz von 90° kann die Kontrolle beispielsweise in Blickrichtung auf eine Oberseite und eine rechte und/oder linke Seite des Körpers erfolgen. Bei einem Winkelversatz von 180° kann die Kontrolle durch gegenüberliegende Seiten des Körpers erfolgen. Sofern mehr als zwei Linsen-Prismen-Paare vorhanden sind, ergibt sich deren Winkelversatz vorzugsweise als Quotient aus 360° Umfangswinkel des Beobachtungskanalabschnitts und der Anzahl der Linsen-Prismen-Paare. Sind beispielsweise zehn Linsen-Prismen-Paare vorhanden, so beträgt deren Winkelversatz vorzugsweise 36°.

In weiterer Ausgestaltung der Erfindung ist ein erstes Linsen-Prismen-Paar an einer ersten Seite, vorzugsweise einer Oberseite, des Körpers und ein zweites Linsen-Prismen-Paar an einer zweiten Seite, vorzugsweise einer gegenüberliegenden Unterseite, des Körpers angeordnet. Diese Ausgestaltung der Erfindung ermöglicht einerseits eine optische Kontrolle unter unterschiedlichen Blickwinkeln. Gleichzeitig kann ein vergleichsweise großer wirksamer Innendurchmesser des Beobachtungskanalabschnitts beibehalten werden. Dabei haben die Erfinder erkannt, dass die Anordnung möglichst vieler winkelversetzter Prismen in dem Beobachtungskanalabschnitt zwar einerseits einen positiven Effekt auf die optische Kontrolle bewirkt. Andererseits wird hierdurch der wirksame Durchmesser des Beobachtungskanalabschnitts verringert. Dies kann zu einer relativ hohen Fließgeschwindigkeit des Flüssigkeitsrückflusses führen, was wiederum einen negativen Effekt auf die optische Kontrolle haben kann. Die Ausgestaltung mit zwei Linsen-Prismen-Paaren bildet insoweit eine Art Optimum.

In weiterer Ausgestaltung der Erfindung weist der Körper im Bereich des Beobachtungskanalabschnitts eine quaderförmige Gestalt mit paarweise gegenüberliegenden Außenseiten auf, wobei die wenigstens eine optische Linse einer der Außenseiten zugeordnet ist. Die quaderförmige Gestalt des Körpers im Bereich des Beobachtungskanalabschnitts ermöglicht, dass letzterer vergleichsweise groß dimensioniert werden kann. Zudem unterstützt die quaderförmige Gestalt die Handhabung des Kanülenansatzes, da die paarweise gegenüberliegenden Außenseiten ergonomisch vorteilhaft zwischen den Fingern einer Hand gegriffen werden können. Überdies lässt die quaderförmige Gestalt eine einfache optische Kontrolle der Winkelausrichtung des Kanülenansatzes zu. Um diese Kontrolle weiter zu vereinfachen, kann der Körper an einer seiner Außenseiten, vorzugsweise seiner Oberseite, einen Markierungsabschnitt aufweisen. Dieser kann beispielsweise in Form einer Aussparung, eines Vorsprungs oder dergleichen gestaltet sein.

In weiterer Ausgestaltung der Erfindung ist der Körper einstückig aus einem transparenten Kunststoff gefertigt. Dies vereinfacht einerseits die Herstellung, da auf unterschiedliche Materialien für unterschiedliche Abschnitte des Körpers verzichtet werden kann. Zum anderen unterstützt die vollständig transparente Gestaltung des Körpers den Lichteinfall in den Beobachtungskanalabschnitt. Hierdurch kann eine nochmals verbesserte optische Kontrolle des Flüssigkeitsrückflusses erfolgen.

Die Erfindung betrifft zudem eine Spinalkanüle mit einem Kanülenansatz nach der vorhergehenden Beschreibung und mit einer an dem Kanülenansatz festgelegten Hohlnadel. Die Hohlnadel ist an dem distalen Ende des Kanülenansatzes festgelegt. Die Hohlnadel ist auf eine dem Fachmann bekannte Weise zwischen einem angeschliffenen distalen Ende und einem proximalen Ende längserstreckt und weist ein durchgängiges Lumen auf. Das Lumen der Hohlnadel ist auf grundsätzlich bekannte Weise fluidleitend mit dem Fluidkanal des Kanülenansatzes verbunden.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Aufsicht eine Ausführungsform einer erfindungsgemäßen Spinalkanüle mit einer Ausführungsform eines erfindungsgemäßen Kanülenansatzes und mit einer an dem Kanülenansatz festgelegten Hohlnadel,
- Fig. 2: eine schematische Perspektivdarstellung des Kanülenansatzes mit Blickrichtung auf ein distales Ende,
- Fig. 3: eine weitere schematische Perspektivdarstellung des Kanülenansatzes mit Blickrichtung auf ein proximales Ende,
- Fig. 4: eine schematische Rückansicht des Kanülenansatzes mit distal gerichteter Blickrichtung,
- Fig. 5: eine schematische Seitenansicht des Kanülenansatzes,
- Fig. 6: eine schematische Längsschnittdarstellung des Kanülenansatzes entlang eines Schnitts VI-VI nach Fig. 5,
- Fig. 7: eine schematische Draufsicht des Kanülenansatzes und
- Fig. 8: eine weitere Längsschnittdarstellung des Kanülenansatzes entlang eines Schnitts VIII-VIII.

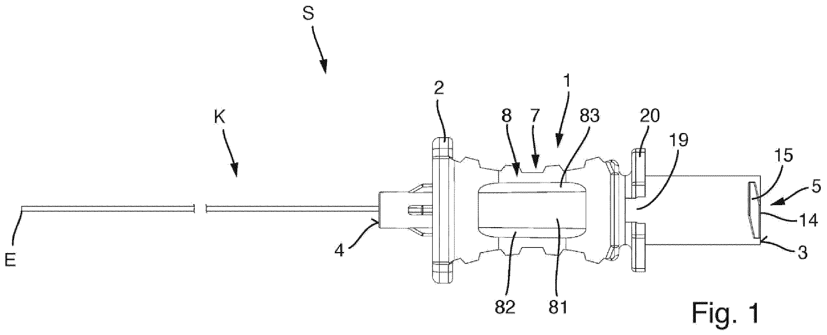

Gemäß Fig. 1 ist eine Spinalkanüle S zur Verwendung bei einer Spinalanästhesie vorgesehen und weist eine Hohlnadel K und einen Kanülenansatz 1 auf.

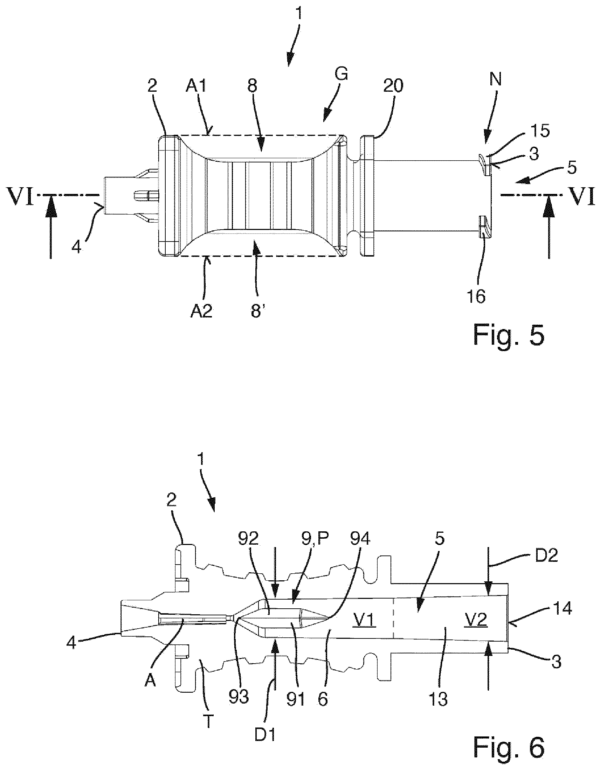

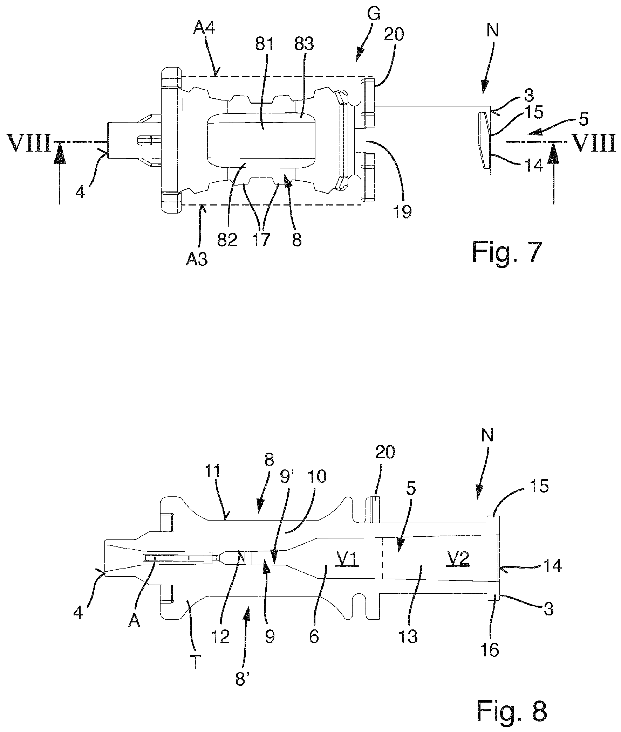

Die Hohlnadel K ist auf eine dem Fachmann bekannte Weise gestaltet und zur Punktion des Rückenmarks vorgesehen. Insoweit ist die Hohlnadel K zwischen einem nicht näher bezeichneten proximalen Ende und einem distalen Ende E längserstreckt, wobei letzteres mit einer angeschliffenen Kanülenspitze versehen ist. Das proximale Ende der Hohlnadel K ist auf eine dem Fachmann bekannte Weise in einer Aufnahmeaussparung A (Fig. 6, 8) des Kanülenansatzes 1 festgelegt. Beispielsweise kann das proximale Ende der Hohlnadel K in die Aufnahmeaussparung A eingeklebt sein.

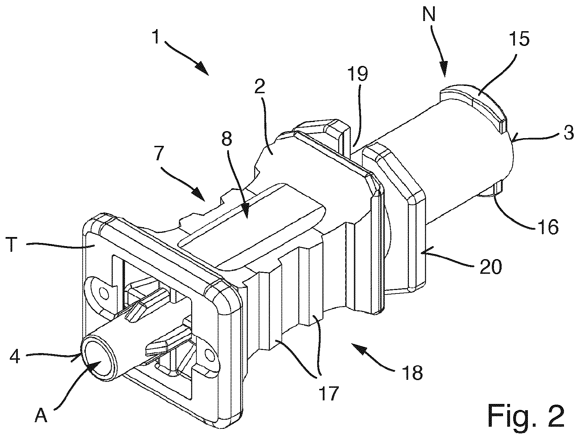

Der Kanülenansatz 1 kann auch als Kanülenhub oder Hub bezeichnet werden und weist einen Körper 2 auf, der zwischen einem proximalen Ende 3 und einem distalen Ende 4 erstreckt ist. Weiter weist der Kanülenansatz 1 einen durch den Körper 2 erstreckten Fluidkanal 5 mit einem Beobachtungskanalabschnitt 6 auf. Der Beobachtungskanalabschnitt 6 ist durch einen transparenten Bereich 7 (Fig. 2) des Körpers 2 hindurch von außen einsehbar. Weiter weist der Kanülenansatz 1 wenigstens eine in dem transparenten Bereich 7 angeordnete optische Linse 8 auf. Mittels der optischen Linse 8 ist der Beobachtungskanalabschnitt 6 optisch vergrößert von außen einsehbar.

In der Anwendung der Spinalkanüle S wird das Rückenmark mittels der Hohlnadel K punktiert. Um die korrekte Lage des distalen Endes E im Bereich des Rückenmarks zu kontrollieren, wird Cerebrospinalflüssigkeit (CSF) durch die Hohlnadel K bis in den Kanülenansatz 1 aspiriert. Sobald sich ein entsprechender Flüssigkeitsrückfluss in proximaler Richtung einstellt, kann von einer korrekten Lage des distalen Endes E ausgegangen werden. Der in dem transparenten Bereich 7 angeordnete Beobachtungskanalabschnitt 6 erlaubt eine optische Kontrolle des besagten Flüssigkeitsrückflusses. Die wenigstens eine optische Linse 8 unterstützt die optische Kontrolle mittels einer optischen Vergrößerung des Beobachtungskanalabschnitts 6.

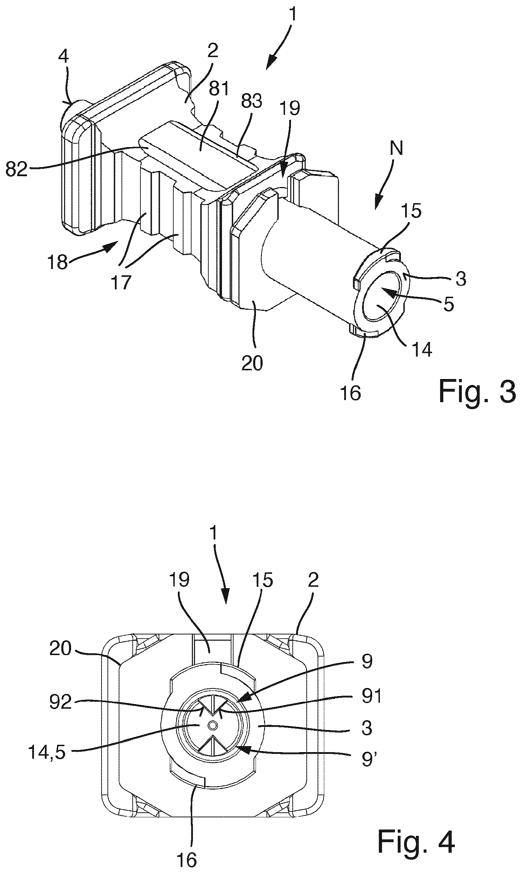

Um eine nochmals verbesserte optische Kontrolle zu ermöglichen, weist der Kanülenansatz 1 zudem wenigstens ein optisches Prisma 9 auf, das anhand der Fig. 4, 6 und 8 gezeigt ist. Das optische Prisma 9 ist in dem Beobachtungskanalabschnitt 6 angeordnet und zur Brechung und/oder Reflexion von durch den transparenten Bereich 7 und/oder die optische Linse 8 hindurch in den Beobachtungskanalabschnitt 6 einfallendem Licht eingerichtet. Das Prisma 9 erlaubt aufgrund seiner das Licht brechenden und/oder reflektierenden Eigenschaften eine deutlich verbesserte optische Kontrolle des Flüssigkeitsrückflusses. Denn je nachdem, ob der Beobachtungskanalabschnitt 6 mit Luft oder Flüssigkeit gefüllt ist, ergeben sich für den Beobachter deutlich unterschiedliche optische Wahrnehmungen. Selbstverständlich ist der Flüssigkeitsrückfluss grundsätzlich auch ohne das Prisma 9 wahrnehmbar. Dies jedoch in deutlich verringertem Umfang. Die vorliegende Kombination aus optischem Prisma 9 und optischer Linse 8 erlaubt selbst unter widrigen optischen Verhältnissen, beispielsweise bei schlechten Lichtverhältnissen, eine zuverlässige Kontrolle des Flüssigkeitsrückflusses.

Nachfolgend werden weitere räumlich-körperliche und funktionelle Merkmale des Kanülenansatzes 1 erläutert. Die nachfolgend erläuterten Merkmale sind ungeachtet ihrer etwaigen Vorteile nicht unbedingt als wesentlich im Hinblick auf die vorliegende Erfindung zu erachten.

Bei der gezeigten Ausführungsform sind die optische Linse 8 und das optische Prisma 9 durch ein- und dieselbe transparente Wandung 10 des Körpers 2 ausgebildet. Dies ist im Detail anhand Fig. 8 gezeigt. Hierdurch sind die optische Linse 8 und die optische Linse 9 einstückig zusammenhängend ausgebildet. Die optische Linse 8 ist einer Außenseite 11 der transparenten Wandung 10 zugeordnet. Das optische Prisma 9 ist einer Innenseite 12 der transparenten Wandung 10 zugeordnet. Die Außenseite 11 ist einer nicht näher bezeichneten Umgebung zugewandt. Die Innenseite 12 ist dem Beobachtungskanalabschnitt 6 zugewandt.

Vorliegend ist der Körper 2 einstückig aus einem transparenten Kunststoff T gefertigt. Insoweit ist der Körper 2 nicht lediglich in dem transparenten Bereich 7 durchsichtig, sondern auch abseits von diesem. Bei zeichnerisch nicht dargestellten Ausführungsformen kann anstelle einer einstückigen Gestaltung des Körpers auch eine mehrteilige Konstruktion vorgesehen sein. Überdies ist der Körper bei weiteren Ausführungsformen ausschließlich in seinem transparenten Bereich durchsichtig gestaltet.

Der Fluidkanal 5 ist zwischen dem proximalen Ende 3 und dem distalen Ende 4 längserstreckt. Ausgehend von dem proximalen Ende 3 weist der Fluidkanal 5 zunächst einen proximalen Kanalabschnitt 13 auf, der in proximaler Richtung in eine proximale Kanalöffnung 14 mündet. In distaler Richtung mündet der proximale Kanalabschnitt 13 in den Beobachtungskanalabschnitt 6. Der Beobachtungskanalabschnitt 6 mündet in distaler Richtung in die Aufnahmeaussparung A. Der Fluidkanal 5 ist im Bereich der zur Illustration in den Fig. 6 und 8 eingezeichneten strichlierten Linie in den proximalen Kanalabschnitt 13 einerseits und den Beobachtungskanalabschnitt 6 andererseits untergliedert. Diese Untergliederung ist als exemplarisch zu verstehen. Jedenfalls ist der Beobachtungskanalabschnitt 6 durch den in der Draufsicht (Fig. 1, 7) unmittelbar unterhalb der optischen Linse 8 erstreckten Abschnitt des Fluidkanals 5 gebildet.

Der Fluidkanal 5 weist ein Gesamtvolumen V1, V2 auf. Das Gesamtvolumen V1, V2 setzt sich bei der gezeigten Ausführungsform aus einem ersten Volumenanteil V1 des Beobachtungskanalabschnitts 6 und einem zweiten Volumenanteil V2 des proximalen Kanalabschnitts 13 zusammen. Mit anderen Worten ergibt sich das Gesamtvolumen V1, V2 vorliegend aus der Summe des ersten Volumenanteils V1 und des zweiten Volumenanteils V2. Bei der gezeigten Ausführungsform ist der erste Volumenanteil V1 des Beobachtungskanalabschnitts 6 möglichst groß dimensioniert. Dies in Relation zu dem zweiten Volumenanteil V2. Bei bevorzugten Ausführungsformen nimmt der erste Volumenanteil V1 einen Großteil des Gesamtvolumens V1, V2 ein. Durch eine möglichst große Dimensionierung des ersten Volumenanteils V1 wird eine relativ niedrige Fließgeschwindigkeit des Flüssigkeitsrückflusses beim Eintreten in den Beobachtungskanalabschnitt 6 erreicht. Dies hat sich aus unterschiedlichen Gründen als besonders vorteilhaft erwiesen.

Weiter mit Bezug auf Fig. 6 weist der Fluidkanal 5 bei der gezeigten Ausführungsform einen maximalen Innendurchmesser D2 auf. Dieser ist vorliegend im Bereich des proximalen Kanalabschnitts 13, genauer: im Bereich der Kanalöffnung 14, verortet. Der Beobachtungskanalabschnitt 6 weist einen maximalen Innendurchmesser D1 auf. Bei der gezeigten Ausführungsform entspricht der maximale Innendurchmesser D1 des Beobachtungskanalabschnitts 6 in etwa dem maximalen Innendurchmesser D2 im Bereich des proximalen Kanalabschnitts 13. Demnach ist der Innendurchmesser, oder hydraulische Durchmesser, des Beobachtungskanalabschnitts 6 in Relation zu dem Innendurchmesser, oder hydraulischen Durchmesser, des Fluidkanals 5 im Übrigen möglichst groß dimensioniert. Dies unterstützt eine Verringerung der Fließgeschwindigkeit des Fluidrückflusses im Bereich des Beobachtungskanalabschnitts 6. Der maximale Innendurchmesser D1 des Beobachtungskanalabschnitts 6 liegt bei der gezeigten Ausführungsform in der anhand Fig. 6 ersichtlichen Längsschnittebene vor. In einer um 90° gedrehten Längsschnittebene gemäß Fig. 8 ergibt sich ein hiervon unterschiedlicher Innendurchmesser. Dies aufgrund des wenigstens einen Prismas 9. Im Hinblick auf die besagte Verringerung der Fließgeschwindigkeit ist eine Verengung in einer Ebene nicht maßgeblich. Entscheidender ist vielmehr, dass der Beobachtungskanalabschnitt 6 einen im Vergleich möglichst großen wirksamen hydraulischen Durchmesser aufweist.

Bei bevorzugten Ausführungsformen ist der maximale hydraulische Durchmesser des Beobachtungskanalabschnitts maximal 45 %, besonders bevorzugt maximal 10 %, kleiner als ein maximaler hydraulischer Durchmesser des proximalen Kanalabschnitts 13.

Der Körper 2 ist im Bereich eines proximalen Endes 3 zur lösbaren Verbindung mit einer fluidführenden medizinischen Komponente, wie beispielsweise einer Spritze, einer medizinischen Schlauchleitung oder dergleichen, eingerichtet. Zu diesem Zweck weist der Körper 2 an seinem proximalen Ende 3 einen genormten Fluidkonnektor N auf. Der Fluidkonnektor N ist vorliegend ein dem Fachmann bekannter NRFit-Konnektor. Dieser weist in Umfangsrichtung des proximalen Endes 3 zueinander winkelversetzt angeordnete Verbindungselemente 15, 16 auf. Die Verbindungselemente 15, 16 sind zur lösbaren formschlüssigen Verbindung mit komplementären Verbindungselementen eines entsprechend komplementären NRFit-Konnektors eingerichtet. Der Fluidkonnektor N entspricht einer Norm DIN EN ISO 80369-6. Dabei umfasst die Normung nicht lediglich die Formgebung der Verbindungselemente 15, 16, sondern auch die Gestaltung des proximalen Kanalabschnitts 13, genauer: dessen Innendurchmesser D2 und/oder Innenkontur. Weiter in Bezug auf Fig. 6 ist erkennbar, dass der Beobachtungskanalabschnitt 6 in proximaler Richtung ohne oder ohne praktisch maßgebliche Querschnittsverringerung in den insoweit maßlich genormten proximalen Kanalabschnitt 13 übergeht. Fertigungsbedingt kann eine Entformschräge von in etwa 1° bis 3° vorhanden sein.

Bei einer zeichnerisch nicht dargestellten Ausführungsform ist der Fluidkonnektor ein Luer-Konnektor, genauer: ein Luer-Lock-Konnektor, nach DIN EN ISO 80369-7. Bei der Ausführungsform mit Luer-Konnektor kann eine geringfügig größere Querschnittsverringerung vorhanden sein.

Die optische Linse 8 weist im Bereich der Außenseite 11 der transparenten Wandung 10 (Fig. 8) unterschiedliche Linsenabschnitte 81, 82, 83 auf. Diese können auch als zentraler Abschnitt 18, medialer Abschnitt 82 und lateraler Abschnitt 83 bezeichnet werden. Der zentrale Abschnitt 81 weist eine ebene Außenkontur auf, so dass insbesondere in Längsrichtung des Beobachtungskanalabschnitts 6 keine Krümmung vorhanden ist. Auch in Querrichtung weist der zentrale Abschnitt 81 vorliegend keine Krümmung auf. Hierzu im Unterschied sind der mediale Abschnitt 82 und der laterale Abschnitt 83 jedenfalls in Querrichtung geneigt und/oder gekrümmt. Die Neigung und/oder Krümmung ist ausgehend von dem zentralen Abschnitt 81 in radialer Richtung des Beobachtungskanalabschnitts 6 nach innen gerichtet.

Die optische Linse 8 ist bei der gezeigten Ausführungsform wenigstens im Wesentlichen, vorzugsweise vollständig, über eine gesamte Länge des Beobachtungskanalabschnitts 6 hinweg längserstreckt. Bei weiteren Ausführungsformen weist die optische Linse 8 eine hierzu unterschiedliche Gestaltung auf.

Das optische Prisma 9 ist bei der gezeigten Ausführungsform als Dachprisma P gestaltet. Das optische Prisma 9 weist eine dreieckige Querschnittsfläche und/oder in radialer Richtung des Beobachtungskanalabschnitts 6 nach innen aufeinander zulaufende Prismenflächen 91, 92 auf, die auch als erste Prismenfläche 91 und zweite Prismenfläche 92 bezeichnet werden können. Die Prismenflächen 91, 92 bilden Begrenzungsflächen des Beobachtungskanalabschnitts 6. Das Prisma 9 weist Stirnenden 93, 94 auf, die einander in Längsrichtung des Beobachtungskanalabschnitts 6 gegenüberliegend angeordnet sind. Im Bereich der Stirnenden 93, 94 ist das Prisma 9 spitzwinklig abgeflacht. Bei zeichnerisch nicht dargestellten Ausführungsformen weist das wenigstens eine Prisma eine unterschiedliche Gestaltung auf. Beispielsweise kann das Prisma als Tripelprisma gestaltet sein.

Bei der gezeigten Ausführungsform entspricht die Längserstreckung des Prismas 9 der Längserstreckung der Linse 8. Das Prisma 9 ist in radialer Richtung des Beobachtungskanals 6 unterhalb des zentralen Abschnitts 81 der Linse 8 angeordnet. In Querrichtung des zentralen Abschnitts 81 ist das Prisma 9 mittig ausgerichtet. Eine gemeinsame Kante der Prismenflächen 91, 92 liegt mittig unterhalb des zentralen Abschnitts 81.

Bei der gezeigten Ausführungsform weist der Kanülenansatz 1 zwei optische Prismen 9, 9' (Fig. 4) und zwei optische Linsen 8, 8' auf (Fig. 5). Diese können auch als erste Linse 8, zweite Linse 8' sowie erstes Prisma 9 und zweites Prisma 9' bezeichnet werden. Die Prismen 9, 9' und die Linsen 8, 8' bilden bei der gezeigten Ausführungsform ein erstes Linsen-Prismen-Paar 8, 9 und ein zweites Linsen-Prismen-Paar 8', 9'.

Hinsichtlich der Gestaltung und Funktionsweise der zweiten Linse 8' und des zweiten Prismas 9' gilt sinngemäß das bereits zu der ersten Linse 8 und dem ersten Prisma 9 Gesagte. Weitere diesbezügliche Erläuterungen sind insoweit entbehrlich.

Das erste Linsen-Prismen-Paar 8, 9 und das zweite Linsen-Prismen-Paar 8', 9' sind in Umfangsrichtung des Beobachtungskanalabschnitts 6 zueinander winkelversetzt angeordnet. Der Winkelversatz beträgt bei der gezeigten Ausführungsform 180°, so dass die besagten Paare in Bezug auf den Beobachtungskanalabschnitt 6 diametral gegenüberliegend angeordnet sind.

Bei einer zeichnerisch nicht dargestellten Ausführungsform ist lediglich ein einziges Linsen-Prismen-Paar vorhanden. Bei weiteren Ausführungsformen sind mehr als zwei, insbesondere drei, vier, fünf, sechs oder mehr Linsen-Prismen-Paare vorhanden. Zudem ist bei einer weiteren Ausführungsform ein Winkelversatz von 90° anstelle der hier gezeigten 180° vorgesehen.

Vorliegend weist der Körper 2 im Bereich des Beobachtungskanalabschnitts 6 eine quaderförmige Gestalt mit paarweise gegenüberliegenden Außenseiten A1, A2 (Fig. 5) sowie A3, A4 auf (Fig. 7). Dabei versteht sich, dass keine exakte quaderförmige Gestalt, sondern lediglich eine dem Grunde nach quaderförmige Gestaltung vorliegt. Die Außenseiten A1 bis A4 können auch als Oberseite A1, Unterseite A2 sowie mediale Seite A3 und laterale Seite A4 bezeichnet werden. Dabei ist das erste Linsen-Prismen-Paar 8, 9 der Oberseite A1 zugeordnet. Das zweite Linsen-Prismen-Paar 8', 9' ist der Unterseite A2 zugeordnet. Die mediale Seite A3 und die laterale Seite A4 sind vorliegend mit jeweils einer mehrere Vorsprünge 17 aufweisenden Riffelung 18 versehen. Die Riffelungen 18 erleichtern ein Greifen und Manipulieren des Kanülenansatzes 1 zwischen den Fingern einer Hand.

Bei der gezeigten Ausführungsform weist der Körper 2 eine Platte 20 auf. Die Platte 20 ist in Längsrichtung des Fluidkanals 5 im Bereich der besagten Untergliederung zwischen dem Beobachtungskanalabschnitt 6 und dem proximalem Kanalabschnitt 13 angeordnet. Die Platte 20 ragt in radialer Richtung nach außen von dem Fluidkonnektor N ab. Die Platte 20 kann beispielsweise als axialer Anschlag für einen komplementären Fluidkonnektor dienen und dementsprechend auch als Anschlagplatte bezeichnet werden. Ob ein Anschlag zustande kommt, hängt selbstverständlich auch von der jeweiligen Gestaltung des komplementären Fluidkonnektors ab.

Der Körper 2 weist ferner einen Markierabschnitt 19 auf, der eine um die Längsachse des Körpers 2 orientierte Ausrichtung markiert. Der Markierabschnitt 19 ist vorliegend der Oberseite A1 zugeordnet. Zudem ist der Markierabschnitt 19 als nicht näher bezeichnete radial nach innen zurückspringende Kerbe an der Platte 20 ausgebildet.

## Patentansprüche

1. Kanülenansatz (1) für eine Spinalkanüle (S), aufweisend
einen zwischen einem proximalen Ende (3) und einem distalen Ende (4) erstreckten Körper (2),
einen durch den Körper (2) erstreckten Fluidkanal (5) mit einem Beobachtungskanalabschnitt (6), der durch einen transparenten Bereich (7) des Körpers (2) hindurch von außen einsehbar ist,
und aufweisend wenigstens eine in dem transparenten Bereich (7) angeordnete optische Linse (8), mittels derer der Beobachtungskanalabschnitt (6) optisch vergrößert einsehbar ist,
**dadurch gekennzeichnet, dass** wenigstens ein optisches Prisma (9) in dem Beobachtungskanalabschnitt (6) vorhanden ist, das zur Brechung und/oder Reflexion von durch die optische Linse (8) hindurch in den Beobachtungskanalabschnitt (6) einfallendem Licht eingerichtet ist.

2. Kanülenansatz (1) nach Anspruch 1, **dadurch gekennzeichnet, dass** die wenigstens eine optische Linse (8) und das wenigstens eine optische Prisma (9) durch ein- und dieselbe transparente Wandung (10) des Körpers (2) ausgebildet und somit einstückig zusammenhängend sind, wobei die wenigstens eine optische Linse (8) einer Außenseite (11) der transparenten Wandung (10) und das wenigstens eine optische Prisma (9) einer Innenseite (12) der transparenten Wandung (10) zugeordnet sind.

3. Kanülenansatz (1) nach Anspruch 1 oder 2, **dadurch gekennzeichnet, dass** der Fluidkanal (5) ein Gesamtvolumen (V1, V2) aufweist, wobei der Beobachtungskanalabschnitt (6) einen überwiegenden Volumenanteil (V1) des Gesamtvolumens (V1, V2) einnimmt.

4. Kanülenansatz (1) nach einem der vorhergehenden Ansprüche, **dadurch gekennzeichnet, dass** der Fluidkanal (5) einen maximalen Innendurchmesser (D2) aufweist, der maximal 45 %, bevorzugt maximal 10 %, größer ist als ein maximaler Innendurchmesser (D1) des Beobachtungskanalabschnitts (6).

5. Kanülenansatz (1) nach einem der vorhergehenden Ansprüche, **dadurch gekennzeichnet, dass** das proximale Ende (3) des Körpers (2) einen genormten Fluidkonnektor (N) mit einem genormten Innendurchmesser (D2) aufweist, wobei ein maximaler Innendurchmesser (D1) des Beobachtungskanalabschnitts (6) maximal 45 %, bevorzugt maximal 10 %, kleiner ist als der genormten Innendurchmesser (D2) des Fluidkonnektors (N).

6. Kanülenansatz (1) nach einem der vorhergehenden Ansprüche, **dadurch gekennzeichnet, dass** wenigstens zwei optische Prismen (9, 9') und wenigstens zwei optische Linsen (8, 8') vorhanden sind, die paarweise als Linsen-Prismen-Paare (8, 9; 8', 9') in Umfangsrichtung des Beobachtungskanalabschnitts (6) zueinander winkelversetzt angeordnet sind.

7. Kanülenansatz (1) nach Anspruch 6, **dadurch gekennzeichnet, dass** ein erstes Linsen-Prismen-Paar (8, 9) an einer ersten Seite (A1), vorzugsweise einer Oberseite, des Körpers (2) und ein zweites Linsen-Prismen-Paar (8', 9') an einer zweiten Seite (A2), vorzugsweise einer gegenüberliegenden Unterseite, des Körpers (2) angeordnet ist.

8. Kanülenansatz (1) nach einem der vorhergehenden Ansprüche, **dadurch gekennzeichnet, dass** der Körper im Bereich des Beobachtungskanalabschnitts (6) eine quaderförmige Gestalt (G) mit paarweise gegenüberliegenden Außenseiten (A1, A2, A3, A4) aufweist, wobei die wenigstens eine optische Linse (8) einer der Außenseiten (A1, A2, A3, A4) zugeordnet ist.

9. Kanülenansatz (1) nach einem der vorhergehenden Ansprüche, **dadurch gekennzeichnet, dass** der Körper (2) einstückig aus einem transparenten Kunststoff (T) gefertigt ist.

10. Spinalkanüle (S) mit einem Kanülenansatz (1) nach einem der vorhergehenden Ansprüche und mit einer an dem Kanülenansatz (1) festgelegten Hohlnadel (K).

## Claims

1. Cannula attachment (1) for a spinal cannula (S), comprising
a body (2) which extends between a proximal end (3) and a distal end (4),
a fluid channel (5) which extends through the body (2) and which has an observation channel section (6) visible from the outside through a transparent region (7) of the body (2),
and comprising at least one optical lens (8) which is arranged in the transparent region (7) and by means of which the observation channel section (6) is visible with optical magnification,
**characterized in that** at least one optical prism (9) is present in the observation channel section (6) and configured for refraction and/or reflection of light incident into the observation channel section (6) through the optical lens (8).

2. Cannula attachment (1) as claimed in claim 1, **characterized in that** the at least one optical lens (8) and the at least one optical prism (9) are formed by the same transparent wall (10) of the body (2) and are thus integrally joined, the at least one optical lens (8) being assigned to an outer face (11) of the transparent wall (10) and the at least one optical prism (9) being assigned to an inner face (12) of the transparent wall (10).

3. Cannula attachment (1) as claimed in claim 1 or 2, **characterized in that** the fluid channel (5) has a total volume (V1, V2), the observation channel section (6) occupying a predominant volume fraction (V1) of the total volume (V1, V2).

4. Cannula attachment (1) as claimed in any of the preceding claims, **characterized in that** the fluid channel (5) has a maximum internal diameter (D2) which is not more than 45%, preferably not more than 10%, greater than a maximum internal diameter (D1) of the observation channel section (6).

5. Cannula attachment (1) as claimed in any of the preceding claims, **characterized in that** the proximal end (3) of the body (2) has a standardized fluid connector (N) having a standardized internal diameter (D2), a maximum internal diameter (D1) of the observation channel section (6) being not more than 45%, preferably not more than 10%, smaller than the standardized internal diameter (D2) of the fluid connector (N).

6. Cannula attachment (1) as claimed in any of the preceding claims, **characterized in that** at least two optical prisms (9, 9') and at least two optical lenses (8, 8'), pairs of which in the form of lens-prism pairs (8, 9; 8', 9') are arranged angularly offset to each other in the circumferential direction of the observation channel section (6), are present.

7. Cannula attachment (1) as claimed in claim 6, **characterized in that** a first lens-prism pair (8, 9) is arranged on a first face (A1), preferably an upper face, of the body (2) and a second lens-prism pair (8', 9') is arranged on a second face (A2), preferably an opposite lower face, of the body (2).

8. Cannula attachment (1) as claimed in any of the preceding claims, **characterized in that** the body in the region of the observation channel section (6) has a cuboid shape (G) with pairs of opposite outer faces (A1, A2, A3, A4), the at least one optical lens (8) being assigned to one of the outer faces (A1, A2, A3, A4).

9. Cannula attachment (1) as claimed in any of the preceding claims, **characterized in that** the body (2) is made of a transparent plastic material (T) in one piece.

10. Spinal cannula (S) comprising a cannula attachment (1) as claimed in any of the preceding claims and comprising a hollow needle (K) fixed to the cannula attachment (1).

## Revendications

1. Embout de canule (1) pour une canule spinale (S), présentant
un corps (2) s'étendant entre une extrémité proximale (3) et une extrémité distale (4),
un canal de fluide (5) s'étendant à travers le corps (2) avec une section de canal d'observation (6) qui peut être vue de l'extérieur à travers une zone transparente (7) du corps (2),
et présentant au moins une lentille optique (8) agencée dans la zone transparente (7), au moyen de laquelle la section de canal d'observation (6) peut être vue agrandie optiquement,
**caractérisé en ce qu'**au moins un prisme optique (9) est présent dans la section de canal d'observation (6), qui est adapté pour réfracter et/ou réfléchir la lumière incidente à travers la lentille optique (8) dans la section de canal d'observation (6).

2. Embout de canule (1) selon la revendication 1, **caractérisé en ce que** l'au moins une lentille optique (8) et l'au moins un prisme optique (9) sont réalisés par une seule et même paroi transparente (10) du corps (2) et sont ainsi solidaires, l'au moins une lentille optique (8) étant associée à un côté extérieur (11) de la paroi transparente (10) et l'au moins un prisme optique (9) étant associé à un côté intérieur (12) de la paroi transparente (10).

3. Embout de canule (1) selon la revendication 1 ou 2, **caractérisé en ce que** le canal de fluide (5) présente un volume total (V1, V2), la section de canal d'observation (6) occupant une fraction volumique prépondérante (V1) du volume total (V1, V2).

4. Embout de canule (1) selon l'une quelconque des revendications précédentes, **caractérisé en ce que** le canal de fluide (5) présente un diamètre intérieur maximal (D2) qui est au maximum 45 %, de préférence au maximum 10 %, plus grand qu'un diamètre intérieur maximal (D1) de la section de canal d'observation (6).

5. Embout de canule (1) selon l'une quelconque des revendications précédentes, **caractérisé en ce que** l'extrémité proximale (3) du corps (2) présente un connecteur de fluide normalisé (N) ayant un diamètre intérieur normalisé (D2), un diamètre intérieur maximal (D1) de la section de canal d'observation (6) étant inférieur d'au maximum 45 %, de préférence d'au maximum 10 %, au diamètre intérieur normalisé (D2) du connecteur de fluide (N).

6. Embout de canule (1) selon l'une quelconque des revendications précédentes, **caractérisé en ce qu'**au moins deux prismes optiques (9, 9') et au moins deux lentilles optiques (8, 8') sont présents, qui sont agencés par paires sous forme de paires lentille-prisme (8, 9 ; 8', 9') décalées angulairement l'une par rapport à l'autre dans la direction circonférentielle de la section de canal d'observation (6).

7. Embout de canule (1) selon la revendication 6, **caractérisé en ce qu'**une première paire lentille-prisme (8, 9) est agencée sur un premier côté (A1), de préférence un côté supérieur, du corps (2) et une deuxième paire lentille-prisme (8', 9') est agencée sur un deuxième côté (A2), de préférence un côté inférieur opposé, du corps (2).

8. Embout de canule (1) selon l'une quelconque des revendications précédentes, **caractérisé en ce que** le corps présente, dans la zone de la section de canal d'observation (6), une forme parallélépipédique (G) avec des côtés extérieurs (A1, A2, A3, A4) opposés par paires, l'au moins une lentille optique (8) étant associée à l'un des côtés extérieurs (A1, A2, A3, A4).

9. Embout de canule (1) selon l'une quelconque des revendications précédentes, **caractérisé en ce que** le corps (2) est fabriqué d'une seule pièce en une matière plastique transparente (T).

10. Canule spinale (S) avec un embout de canule (1) selon l'une quelconque des revendications précédentes et avec une aiguille creuse (K) fixée à l'embout de canule (1) .
